(19) Europäisches Patentamt
European Patent Office
Office européen des brevets

(11) Veröffentlichungsnummer : **0 070 012**
**B1**

(12) # EUROPÄISCHE PATENTSCHRIFT

(45) Veröffentlichungstag der Patentschrift : **21.10.87**

(51) Int. Cl.[4] : **C 07 D213/09**, C 07 D213/06

(21) Anmeldenummer : **82106171.0**

(22) Anmeldetag : **09.07.82**

(54) **Verfahren zur Herstellung von 3,5-Dialkylpyridinen.**

(30) Priorität : **09.07.81 CH 4504/81**

(43) Veröffentlichungstag der Anmeldung : **19.01.83 Patentblatt 83/03**

(45) Bekanntmachung des Hinweises auf die Patenterteilung : **21.10.87 Patentblatt 87/43**

(84) Benannte Vertragsstaaten : **AT BE CH DE FR GB IT LI LU NL SE**

(56) Entgegenhaltungen :
**EP-A- 0 040 698**
**DE-A- 1 695 296**
**DE-A- 2 045 884**
**DE-A- 2 045 885**

(73) Patentinhaber : **LONZA AG**
**Gampel/Wallis (CH)**

(72) Erfinder : **Grayson, James Ian, Dr.**
**Bürchnerstrasse 11**
**Visp (Kanton Wallis) (CH)**

(74) Vertreter : **Weinhold, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40 (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein Verfahren zur Herstellung von 3,5-Dialkylpyridinen.

3,5-Dialkylpyridine stellen wichtige Zwischenprodukte in der chemischen Industrie dar, so z. B. bei der Herstellung von Pflanzenschutzmitteln oder Dinikotinsäure. Es sind verschiedene Verfahren zur Herstellung von 3,5-Dialkylpyridinen bekannt.

Es ist bekannt, 3,5-Dialkylpyridine in einer Gasphasenreaktion aus Formaldehyd, Ammoniak und einem aliphatischen Aldehyd der Formel $R—CH_2—CHO$ herzustellen (FR-A-1 332 908). So wurde aus Formaldehyd, n-Butyraldehyd und Ammoniak bei 400 °C an einem mit Bleifluorid imprägnierten Aluminiumsilikat-Katalysator 3,5-Diäthylpyridin erhalten. Die Ausbeute entspricht einem Umsatz von 50 %. Analog wurde aus Formaldehyd, Propionaldehyd und Ammoniak 3,5-Dimethylpyridin erhalten. Das Verfahren hat bislang keine technische Bedeutung erlangt. Nachteilig ist vor allem die Notwendigkeit, den Katalysator im Sauerstoff- oder Luftstrom immer wieder regenerieren zu müssen. Bei den erforderlichen hohen Temperaturen bildet sich teeriges Material und dessen Zersetzungsprodukte, die zur Verkokung der Apparatur und des Katalysators Anlass geben und die Abtrennung des gewünschten 3,5-Dialkylpyridins erschweren.

Aus der DE-A-2 123 965 ist ein zweistufiges Flüssigphasenverfahren bekannt, nach dem in der ersten Stufe ein Vorkondensat aus Formaldehyd und einem Aldehyd $R—CH_2—CHO$ gebildet wird, das anschliessend in einer zweiten Stufe mit Ammoniak und/oder Ammoniumsalzen bei 220 bis 300 °C während 8 bis 10 Stunden zum 3,5-Dialkylpyridin übergeführt wird. Die Ausbeuten liegen zwischen 11 und 46 %. Der Nachteil dieses Verfahrens liegt darin, dass es zweistufig ist und lange Reaktionszeiten benötigt.

Aus der DE-A-2 045 885 ist die Herstellung von 3,5-Dialkylpyridin über ein zweistufiges Verfahren bekannt. Hierbei wird ein Vorkondensat gebildet aus Formaldehyd, höheren Aldehyden der Formel $RCH_2CHO$ und Aminen der Formel $R_3N$ in Gegenwart von Wasser/niedrigen Alkoholen in der Flüssigphase bei 180 bis 350 °C und unter erhöhtem Druck, gegebenenfalls in Anwesenheit des bei der Vorkondensatherstellung verwendeten (sauren) Katalysators. Das Verfahren umfaßt eine separate Vorkondensatherstellung und eine anschliessende Kondensation. Die Beispiele für dieses Verfahren zeigen in besonderer Weise nachteilig sehr lange Reaktionszeiten und dazu vergleichsweise niedrige Ausbeuten an 3,5-Dialkylpyridin.

Die DE-A-1 695 296 beinhaltet ein Verfahren zur Herstellung von Pyridinen aus Aldehyden der Formel $RCH_2CHO$ und einem Ammoniumsalz. Die Reaktion findet in Lösung in Gegenwart einer freien Säure und zweckmäßig in Gegenwart von molekularem Sauerstoff und Cu-, Mn-, oder Co-Salzen statt. Das Verfahren bedient sich teurer Katalysatorsalze und einer schwierigen Verfahrenstechnik. Ziel der vorliegenden Erfindung ist es, 3,5-Dialkylpyridine in hohen Ausbeuten und in kurzen Reaktionszeiten herzustellen. Vorzugsweise können so 3,5-Dialkylpyridine mit 1 bis 4 Kohlenstoffatomen in jeder Alkylgruppe, insbesondere 3,5-Lutidin und 3,5-Diäthylpyridin hergestellt werden.

Verfahren zur Herstellung von 3,5-Dialkylpyridinen durch Umsetzung von Aldehydgemischen aus Aldehyden der Formel

$$R—CH_2—CHO$$

worin R = ein geradekettiger oder verzweigter Alkylrest mit 1 bis 6 Kohlenstoffatomen bedeutet, und einer Formaldehydverbindung in flüssiger wässriger Phase unter Druck, bei erhöhter Temperatur, mit einer Stickstoffenthaltenden Verbindung, dadurch gekennzeichnet, daß, ohne Vorkondensatbildung der Aldehyde, direkt ein Gemisch des Aldehyds der Formel $R—CH_2—CHO$ oder deren Acetale, worin R die oben genannte Bedeutung besitzt, mit Formaldehyd und/oder Hexamethyltetramin und/oder ein Formaldehydacetal bei Temperaturen von 180 bis 280 °C im geschlossenen Gefäß entweder in Gegenwart von Ammoniak und/oder von wasserlöslichen Ammoniumsalzen der Phosphorsäure oder Essigsäure, oder in Gegenwart von Alkalisalzen der Phosphorsäure oder Schwefelsäure zusammen mit dem Ammoniak umgesetzt wird.

Unter Aldehyden der Formel $R—CH_2—CHO$ im Sinne der Erfindung können auch deren Acetale, wie z. B. Propionaldehyddiäthylacetal, verstanden werden ; unter Formaldehyd fallen auch dessen Acetale, wie z. B. Formaldehyddimethylacetal, und dessen Polymere, wie z. B. Trioxan. Bei vorteilhaft verwendbaren Acetalen handelt es sich z. B. um Nieder-Alkyl-Acetale wie Acetaldehydmethyl- oder -äthylacetal bzw. Formaldehydmethyl- oder -äthylacetal.

Wasserlösliche Alkali- oder Ammoniumsalze im Sinne vorliegender Erfindung sind beispielsweise die folgenden : Kaliumdihydrogenphosphat, Ammoniumdihydrogenphosphat, Dikaliumhydrogenphosphat, Diammoniumhydrogenphosphat, Natriumhydrogensulfat oder Ammoniumacetat.

Der Ammoniak kann entweder gasförmig oder als wässrige Lösung eingesetzt werden.

Werden miteinander nicht mischbare flüssige Ausgangsmaterialien zusammen mit wässrigem Formaldehyd verwendet, so ist es vorteilhaft, zur Homogenisierung kleine Mengen Homogenisierungsmittel, wie Alkohole oder cyclische Aether, einzusetzen. Das Verfahren der Erfindung wird vorteilhaft mit einem Molverhältnis Aldehyd zu Formaldehyd und/oder Hexamethylentetramin und/oder Formaldehydacetal von 1 zu 0,5 bis 1 zu 1,5, vorzugsweise von 1 zu 0,8 bis 1 zu 1,2, durchgeführt.

Die Reaktionstemperaturen liegen bei 180 bis

280 °C, insbesondere bei 205 bis 240 °C, vorzugsweise bei 225 bis 235 °C.

Die Reaktion wird in flüssiger Phase (wässriger Phase) unter einem Druck, der sich bei der Reaktion im geschlossenen Gefäss bei vorgegebener Temperatur einstellt, durchgeführt. Es ist vorteilhaft, während der Reaktion den Reaktionssatz zu rühren.

Die Mengen an Ammoniak und/oder Ammoniumionen liegen bei 0,5 bis 3 Mol Ammoniak und/oder Ammoniumionen pro Mol Aldehyd, zweckmässig bei 0,5 bis 2,0 Mol pro Mol Aldehyd.

Die Mengen an Säureanionen liegen vorteilhaft bei 0,1 bis 3 Mol, vorzugsweise bei 0,2 bis 1,0 Mol pro Mol Aldehyd.

Der Start-pH-Wert der wässrigen Reaktionslösung liegt vorteilhaft zwischen 5,0 und 12,5.

Die Zugabe des Aldehydes erfolgt vorteilhaft nach Massgabe seines Verbrauches. So ist es beispielsweise günstig, beim Arbeiten in einem 2-Liter-Behälter und bei Einsatz von 350 ml Aldehyd diesen kontinuierlich während 30 bis 90 Minuten zuzusetzen. Bei anderen Bedingungen sind die entsprechenden Zugabezeiten zu wählen.

Am Ende der gewünschten Reaktionsperiode wird die Temperatur auf etwa Raumtemperatur gesenkt und das 3,5-Dialkylpyridin auf bekannte Weise aus der Reaktionsmischung gewonnen. Eine Methode besteht darin, dass man den pH-Wert der Wasserphase zuerst in den basischen Bereich bringt und dann das organische Material aus der wässrigen Reaktionsmischung mit einem organischen Lösungsmittel, wie z. B. Benzol, Toluol, Xylol, Methylenchlorid, Chloroform, Aether und dergleichen, extrahiert. Das organische Lösungsmittel wird dann abgedampft und man erhält 3,5-Dialkylpyridin durch fraktionierte Destillation. Im Rahmen der Erfindung können auch beliebige andere Methoden zur Abtrennung bzw. Aufarbeitung des Produktes angewendet werden.

Ein Vorteil des neuen erfindungsgemässen Verfahrens ist auch der, dass die nach der Extraktion der Reaktionsmischung mit einem organischen Lösungsmittel erhaltene wässrige Phase nach Wiederanreicherung mit Ammoniak und/oder Ammoniumionen in den Reaktor zurückgeführt werden kann. Die wässrige Salzphase ist zusammengesetzt aus der ursprünglich in der Salzlösung vorhandenen Wassermenge, der nicht umgesetzten Menge an Ammoniak und/oder Ammoniumsalz, dem gegebenenfalls vorhandenen Metallsalz, der freigesetzten Säure des an der Umsetzung teilnehmenden Ammoniumsalzes sowie einem Mol Wasser für jedes Mol bei der Umsetzung verbrauchten Aldehyds. Die wässrige Salzphase wird daher mit Hilfe eines beliebigen bekannten verfahrens konzentriert, z. B. durch Verdampfen, um das infolge der Kondensationsreaktion gebildete Wasser zu entfernen.

Eine Wiederanreicherung von Ammoniak und/oder Ammoniumsalz wird dann dadurch erreicht, dass man in die wässrige Lösung bei Umgebungstemperatur gasförmiges Ammoniak einbringt, unter Rückbildung des Ammoniumsalzes aus der gegebenenfalls vorhandenen Säure.

Obgleich die Erfindung als diskontinuierliches Verfahren beschrieben worden ist, kann das Verfahren auch im Rahmen der vorliegenden Erfindung kontinuierlich betrieben werden. Bei einer Ausführungsform eines kontinuierlichen Verfahrens werden die Reaktionsteilnehmer kontinuierlich in einen geeigneten Druckreaktor eingeführt, aus dem die Reaktionsmischung kontinuierlich abgezogen wird. Die Reaktionsprodukte werden daraus abgetrennt, die wässrige Salzphase aufkonzentriert und verbrauchte Reaktionsteilnehmer werden dann wieder ergänzt und in das Reaktionsgefäss zurückgeführt.

Das kontinuierliche Verfahren kann in jedem Reaktor durchgeführt werden, der eine innige Vermischung der Reaktionsteilnehmer unter heftigem Rühren gestattet, z. B. in einem kontinuierlich gerührten Tankreaktor.

Beispiel 1

1 140 ml einer 3,40 molaren wässrigen Lösung von Diammoniumhydrogenphosphat (pH 8,3) wurden in einem 2-Liter-Autoklaven auf 230 °C erhitzt und bei 1 500 UpM gerührt. In diese Lösung wurde innert 62 Minuten kontinuierlich ein Gemisch aus 127,6 g Propionaldehyd, 213,3 g einer 30,2 %igen Formaldehydlösung und 50,0 g Aethanol eingepumpt (Molverhältnis Propionaldehyd zu Formaldehyd = 1 zu 1). Dabei variierte der Reaktionsdruck zwischen 31 und 42 bar. Nach beendeter Zugabe des Eduktgemisches wurde die Reaktionsmasse während 10 Minuten bei 230 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte. Die auf eingesetzten Propionaldehyd bezogene Ausbeute an 3,5-Lutidin betrug 46,1 %.

Sämtliche gaschromatographischen Analysen wurden unter Verwendung eines internen Standards sowie unter Berücksichtigung von Flächenkorrekturfaktoren durchgeführt.

Beispiel 2

1 140 ml einer 3,40 molaren wässrigen Lösung von Diammoniumhydrogenphosphat (pH 8,3) wurden in einem 2-Liter-Autoklaven auf 210 °C erhitzt und bei 1 500 UpM gerührt. In diese Lösung wurde innert 56 Minuten kontinuierlich ein Gemisch aus 127,6 g Propionaldehyd, 213,3 g einer 30,3 %igen Formaldehydlösung und 50,0 g Aethanol eingepumpt (Molverhältnis Propionaldehyd zu Formaldehyd = 1 zu 1). Dabei variierte der Reaktionsdruck zwischen 21 und 30 bar. Nach beendeter Zugabe des Eduktgemisches wurde die Reaktionsmasse während 10 Minuten bei 210 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Propionaldehyd bezogene

Ausbeute an 3,5-Lutidin betrug 38,3 %.

Beispiel 3

1 140 ml einer 3,40 molaren wässrigen Lösung von Diammoniumhydrogenphosphat (pH 8,3) wurden in einem 2-Liter-Autoklaven auf 232 °C erhitzt und bei 1 500 UpM gerührt. In diese Lösung wurde innert 58 Minuten kontinuierlich ein Gemisch aus 148,7 g Butyraldehyd, 201,0 g einer 30,2 %igen Formaldehydlösung und 70,0 g Aethanol eingepumpt (Molverhältnis Butyraldehyd zu Formaldehyd = 1 zu 1). Dabei variierte der Reaktionsdruck zwischen 33 und 47 bar. Nach beendeter Zugabe des Eduktgemisches wurde die Reaktionsmasse während 20 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Butyraldehyd bezogene Ausbeute an 3,5-Diäthylpyridin betrug 55,0 %.

Beispiel 4

1 140 ml einer 3,40 molaren wässrigen Lösung von Diammoniumhydrogenphosphat (pH 8,3) wurden in einem 2-Liter-Autoklaven auf 210 °C erhitzt und bei 1 500 UpM gerührt. In diese Lösung wurde innert 55 Minuten kontinuierlich ein Gemisch aus 148,7 g Butyraldehyd, 200,5 g einer 30,3 %igen Formaldehydlösung und 59,0 g Aethanol eingepumpt (Molverhältnis Butyraldehyd zu Formaldehyd = 1 zu 1). Dabei variierte der Reaktionsdruck zwischen 21 und 33 bar. Nach beendeter Zugabe des Eduktgemisches wurde die Reaktionsmasse während 10 Minuten bei 210 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Butyraldehyd bezogene Ausbeute an 3,5-Diäthylpyridin betrug 47,1 %.

Beispiel 5

1 140 ml einer 3,40 molaren wässrigen Lösung von Diammoniumhydrogenphosphat (pH 8,3) wurden in einem 2-Liter-Autoklaven auf 232 °C erhitzt und bei 1 500 UpM gerührt. In diese Lösung wurde innert 58 Minuten kontinuierlich ein Gemisch von 148,7 g Butyraldehyd, 47,9 g Hexamethylentetramin, 120 g Wasser und 110 g Aethanol eingepumpt (kalkulatorisches Molverhältnis Butyraldehyd zu Formaldehyd = 1 zu 1). Dabei variierte der Reaktionsdruck zwischen 32 und 58 bar. Nach beendeter Zugabe des Eduktgemisches wurde die Reaktionsmasse während 20 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Butyraldehyd bezogene Ausbeute an 3,5-Diäthylpyridin betrug 49,0 %.

Beispiel 6

1 140 ml einer 3,40 molaren wässrigen Lösung von Ammoniumacetat (pH 7,7) wurden in einem 2-Liter-Autoklaven auf 232 °C erhitzt und bei 1 500 UpM gerührt. In diese Lösung wurde innert 57 Minuten kontinuierlich ein Gemisch aus 127,6 g Propionaldehyd, 213,3 g einer 30,3 %igen Formaldehydlösung und 50,0 g Aethanol eingepumpt (Molverhältnis Propionaldehyd zu Formaldehyd = 1 zu 1). Dabei variierte der Reaktionsdruck zwischen 26 und 34 bar. Nach beendeter Zugabe des Eduktgemisches wurde die Reaktionsmasse während 10 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Propionaldehyd bezogene Ausbeute an 3,5-Lutidin betrug 44,2 %.

Beispiel 7

Eine Lösung von 397,1 g Dikaliumhydrogenphosphat in 1 140 ml 2,5 molarem wässrigem Ammoniak (pH 12,0) wurde in einem 2-Liter-Autoklaven auf 232 °C erhitzt und bei 1 500 UpM gerührt. In diese Lösung wurde innert 60 Minuten kontinuierlich ein Gemisch aus 127,6 g Propionaldehyd, 213,3 g einer 30,1 %igen Formaldehydlösung und 50,0 g Aethanol eingepumpt (Molverhältnis Propionaldehyd zu Formaldehyd = 1 zu 1). Dabei variierte der Reaktionsdruck zwischen 33 und 35 bar. Nach beendeter Zugabe des Eduktgemisches wurde die Reaktionsmasse während 10 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Propionaldehyd bezogene Ausbeute an 3,5-Lutidin betrug 28,8 %.

Beispiel 8

1 140 ml einer 3,40 molaren wässrigen Lösung von Diammoniumhydrogenphosphat (pH 8,3) wurden in einem 2-Liter-Autoklaven auf 232 °C erhitzt und bei 1 500 UpM gerührt. In diese Lösung wurde innert 64 Minuten kontinuierlich ein Gemisch aus 140,0 g Butyraldehyd und 153,0 g Formaldehyddimethylacetal eingepumpt (Molverhältnis Butyraldehyd zu Formaldehyddimethylacetal = 1 zu 1). Dabei variierte der Reaktionsdruck zwischen 33 und 43 bar. Nach beendeter Zugabe des Eduktgemisches wurde die Reaktionsmasse während 20 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Butyraldehyd bezogene Ausbeute an 3,5-Diäthylpyridin betrug 46,7 %.

Beispiel 9

Eine Lösung von 137,0 g Natriumhydrogensulfatmonohydrat in 1 140 ml 4,0 molarem wässrigem Ammoniak (pH 10,8 wurde in einem 2-Liter-Autoklaven auf 232 °C erhitzt und bei 1 500 UpM gerührt. In diese Lösung wurde innert 64 Minuten kontinuierlich ein Gemisch aus 140,0 g Butyraldehyd und 153,0 g Formaldehyddimethylacetal eingepumpt (Molverhältnis Butyraldehyd zu Formaldehyddimethylacetal = 1 zu 1). Dabei variierte der Reaktionsdruck zwischen 36 und 44 bar. Nach beendeter Zugabe des Eduktgemisches wurde die Reaktionsmasse während 20 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Butyraldehyd bezogene Ausbeute an 3,5-Diäthylpyridin betrug 58,6 %.

Beispiel 10

1 140 ml einer 3,40 molaren wässrigen Lösung von Diammoniumhydrogenphosphat (pH 8,3) wurde in einem 2-Liter-Autoklaven auf 232 °C erhitzt und bei 1 500 UpM gerührt. In diese Lösung wurde innert 74 Minuten kontinuierlich ein Gemisch aus 127,6 g Propionaldehyd, 179,0 g einer 30,1 %igen Formaldehydlösung und 50,0 g Aethanol eingepumpt (Molverhältnis Propionaldehyd zu Formaldehyd = 1,2 zu 1). Dabei variierte der Reaktionsdruck zwischen 33 und 40 bar. Nach beendeter Zugabe des Eduktgemisches wurde die Reaktionsmasse während 10 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Propionaldehyd bezogene Ausbeute an 3,5-Lutidin betrug 47,4 %.

Beispiel 11

1 140 ml einer 3,40 molaren wässrigen Lösung von Diammoniumhydrogenphosphat (pH 8,3) wurden in einem 2-Liter-Autoklaven auf 232 °C erhitzt und bei 1 500 UpM gerührt. In diese Lösung wurde innert 62 Minuten kontinuierlich ein Gemisch aus 105,6 g Propionaldehyd, 213,3 g einer 30,1 %igen Formaldehydlösung und 50,0 g Aethanol eingepumpt (Molverhältnis Propionaldehyd zu Formaldehyd = 0,83 zu 1). Dabei variierte der Reaktionsdruck zwischen 33 und 47 bar. Nach beendeter Zugabe des Eduktgemisches wurde die Reaktionsmasse während 10 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Propionaldehyd bezogene Ausbeute an 3,5-Lutidin betrug 47,5 %.

Beispiel 12

1 140 ml einer 3,40 molaren wässrigen Lösung von Diammoniumhydrogenphosphat (pH 8,3) wurden in einem 2-Liter-Autoklaven auf 232 °C erhitzt und bei 1 500 UpM gerührt. In diese Lösung wurde innert 63 Minuten kontinuierlich ein Gemisch aus 127,6 g Propionaldehyd, 143,6 g einer 30,1 %igen Formaldehydlösung und 50,0 g Aethanol eingepumpt (Molverhältnis Propionaldehyd zu Formaldehyd = 1,5 zu 1). Dabei variierte der Reaktionsdruck zwischen 33 und 39 bar. Nach beendeter Zugabe des Eduktgemisches wurde die Reaktionsmasse während 10 Minuten bei 232 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Propionaldehyd bezogene Ausbeute an 3,5-Lutidin betrug 48,5 %.

Beispiel 13

1 140 ml einer 3,40 molaren wässrigen Lösung von Diammoniumhydrogenphosphat (pH 8,3) wurden in einem 2-Liter-Autoklaven auf 230 °C erhitzt und bei 1 500 UpM gerührt. In diese Lösung wurde innert 64 Minuten kontinuierlich ein Gemisch aus 202,3 g Propionaldehyddiäthylacetal und 120,2 g Formaldehyddimethylacetal eingepumpt (Molverhältnis Propionaldehyddiäthylacetal zu Formaldehyddimethylacetal = 1 zu 1). Dabei variierte der Reaktionsdruck zwischen 32 und 46 bar. Nach beendeter Zugabe des Eduktgemisches wurde die Reaktionsmasse während 10 Minuten bei 230 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Propionaldehyd bezogene Ausbeute an 3,5-Lutidin betrug 48,6 %.

Beispiel 14

1 140 ml einer 3,40 molaren wässrigen Lösung von Diammoniumhydrogenphosphat (pH 8,3) wurden in einem 2-Liter-Autoklaven auf 230 °C erhitzt und bei 1 500 UpM gerührt. In diese Lösung wurde innert 61 Minuten kontinuierlich ein Gemisch aus 88,9 g Propionaldehyd, 46,0 g Trioxan, 100,0 g Wasser und 50,0 g Aethanol eingepumpt (kalkulatorisches Molverhältnis Propionaldehyd zu Formaldehyd = 1 zu 1). Dabei variierte der Reaktionsdruck zwischen 32 und 37 bar. Nach beendeter Zugabe des Eduktgemisches wurde die Reaktionsmasse während 10 Minuten bei 230 °C weitergerührt und dann auf Raumtemperatur abgekühlt. Schliesslich erfolgte eine Extraktion mit 3 mal 100 ml Methylenchlorid sowie eine gaschromatographische Analyse der vereinigten Methylenchloridextrakte.

Die auf eingesetzten Propionaldehyd bezogene Ausbeute an 3,5-Lutidin betrug 38,1 %.

Beispiel 15

1 140 ml einer 3,40 molaren wässrigen Lösung von Diammoniumhydrogenphosphat (pH 8,3) wurden in einem 2-Liter-Autoklaven auf 232 °C erhitzt und bei 1 500 UpM gerührt. In diese Lösung wurde innert 63 Minuten kontinuierlich ein Gemisch aus 150,7 g Isovaleraldehyd, 174,6 g einer 30,1 %igen Formaldehydlösung und 115 g Aethanol eingepumpt (Molverhältnis Isovaleraldehyd zu Formaldehyd = 1 zu 1). Dabei variierte der Reaktionsdruck zwischen 32 und 49 bar. Nach beendeter Zugabe des Eduktgemisches wurde die Reaktionsmasse während 20 Minuten bei 232 °C weitergerührt und dann auf Raumtemperatur abgekühlt. Schliesslich erfolgte eine Extraktion mit 3 mal 100 ml Methylenchlorid. Die vereinigten Methylenchloridextrakte aus 3 solchen Versuchen wurden destilliert, wobei 226,6 g 3,5-Diisopropylpyridin erhalten wurden. Die gaschromatographische Analyse ergab einen Gehalt von 96,1 %, der einer Ausbeute an 3,5-Diisopropylpyridin von 51,3 % entsprach.

Beispiel 16

1 140 ml einer 3,40 molaren wässrigen Lösung von Diammoniumhydrogenphosphat (pH 8,3) wurden in einem 2-Liter-Autoklaven auf 232 °C erhitzt und bei 1 500 UpM gerührt. In diese Lösung wurde innert 63 Minuten kontinuierlich ein Gemisch aus 151,0 g Capronaldehyd, 150,0 g einer 30,1 %igen Formaldehydlösung und 130,0 g Aethanol eingepumpt (Molverhältnis Capronaldehyd zu Formaldehyd = 1 zu 1). Dabei variierte der Reaktionsdruck zwischen 33 und 46 bar. Nach beendeter Zugabe des Eduktgemisches wurde die Reaktionsmasse während 20 Minuten bei 232 °C weitergerührt und dann auf Raumtemperatur abgekühlt. Schliesslich erfolgte eine Extraktion mit 3 mal 100 ml Methylenchlorid. Die vereinigten Methylenchloridextrakte aus 3 solchen Versuchen wurden destilliert, wobei 217,3 g 3,5-Dibutylpyridin erhalten wurden. Die gaschromatographische Analyse ergab einen Gehalt von 92,3 %, der einer Ausbeute an 3,5-Dibutylpyridin von 46,9 % entsprach.

**Patentansprüche**

1. Verfahren zur Herstellung von 3,5-Dialkylpyridinen durch Umsetzung von Aldehydgemischen aus Aldehyden der Formel

$$R—CH_2—CHO$$

worin R = ein geradekettiger oder verzweigter Alkylrest mit 1 bis 6 Kohlenstoffatomen bedeutet, und einer Formaldehydverbindung in flüssiger wässriger Phase unter Druck, bei erhöhter Temperatur, mit einer Stickstoffenthaltenden Verbindung, dadurch gekennzeichnet, daß, ohne Vorkondensatbildung der Aldehyde, direkt ein Gemisch des Aldehyds der Formel $R—CH_2—CHO$ oder dessen Acetale, worin R die oben genannte Bedeutung besitzt, mit Formaldehyd und/oder Hexamethylentetramin und/oder einem Formaldehydacetal bei Temperaturen von 180 bis 280 °C im geschlossenen Gefäß entweder in Gegenwart von Ammoniak und/oder von wasserlöslichen Ammoniumsalzen der Phosphorsäure oder Essigsäure, oder in Gegenwart von Alkalisalzen der Phosphorsäure oder Schwefelsäure zusammen mit dem Ammoniak umgesetzt wird.

2. Verfahren gemäß Patentanspruch 1, dadurch gekennzeichnet, daß man ein Molverhältnis Aldehyd der Formel $R—CH_2—CHO$ zu Formaldehyd und/oder Hexamethylentetramin und/oder Formaldehydacetal von 1 zu 0,5 bis 1 zu 1,5 anwendet.

3. Verfahren gemäß Patentansprüchen 1 bis 2, dadurch gekennzeichnet, daß man bei Temperaturen von 205 bis 240 °C arbeitet.

4. Verfahren gemäß Patentansprüchen 1 bis 3, dadurch gekennzeichnet, daß man die Salze in wässriger Lösung in einer Konzentration von 0,3 bis 10 Mol/l anwendet.

5. Verfahren gemäß Patentansprüchen 1 bis 4, dadurch gekennzeichnet, daß man die Aldehyde der Formel $R—CH_2—CHO$ in Form deren Acetale verwendet.

**Claims**

1. Process for the production of 3,5-dialkyl pyridines by the reaction of aldehyde mixtures of aldehydes having the formula

$$R—CH_2—CHO$$

in which R is a straight chain or branched alkyl group having 1 to 6 carbon atoms, and a formaldehyde compound in liquid aqueous phase under pressure at elevated temperature with a nitrogen containing compound, characterized in that without formation of a precondensate of the aldehydes a mixture of the aldehyde having the formula $R—CH_2—CHO$ or its acetals wherein R has the above mentioned meaning with formaldehyde and/or hexamethylentetramine and/or a formaldehyde acetal is directly reacted in a closed vessel at temperatures of 180 to 280 °C either in the presence of ammonia and/or of water-soluble ammonium salts of phosphoric acid or acetic acid, or in the presence of alkali salts of phosphoric acid or sulfuric acid together with the ammonia.

2. Process according to patent claim 1, characterized in that a molar ratio of the aldehyde having the formula $R—CH_2—CHO$ to formaldehyde and/or hexamethylentetramine and/or formaldehyde acetal of 1 : 0.5 to 1 : 1.5 is used.

3. Process according to patent claims 1 to 2, characterized in that one operates at temperatures of 205 to 240 °C.

4. Process according to patent claims 1 to 3, characterized in that the salts are used in aqueous solution in a concentration of 0.3 to 10 mole/l.

5. Process according to patent claims 1 to 4, characterized in that the aldehydes having the formula R—$CH_2$—CHO are used in the form of their acetals.

**Revendications**

1. Procédé pour la préparation de 3,5-dialkylpyridines par conversion de mélanges d'aldéhydes constitués d'aldéhydes de formule

$$R—CH_2—CHO$$

dans laquelle R est un groupe alkyle linéaire ou ramifié avec 1 à 6 atomes de carbone, et d'un composé du formaldéhyde en phase liquide aqueuse sous pression, à température élevée, avec un composé contenant de l'azote, caractérisé en ce qu'on fait réagir directement ensemble avec l'ammoniac un mélange de l'aldéhyde de formule R—$CH_2$—CHO ou de ses acétals, R ayant la signification donnée ci-dessus, et de formaldéhyde et/ou d'hexaméthylène tétramine et/ou d'un acétal de formaldéhyde sans formation d'un précondensat des aldéhydes, à des températures de 180 à 280 °C en récipient fermé, soit en présence d'ammoniac et/ou de sels d'ammonium hydrosolubles de l'acide phosphorique ou de l'acide acétique, soit en présence de sels alcalins de l'acide phosphorique ou de l'acide sulfurique.

2. Procédé selon la revendication 1, caractérisé en ce que l'on utilise un rapport molaire de l'aldéhyde de formule R—$CH_2$—CHO au formaldéhyde et/ou à l'hexaméthylènetétramine et/ou à l'acétal de formaldéhyde de 1 à 0,5 jusqu'à 1 à 1,5.

3. Procédé selon les revendications 1 et 2, caractérisé en ce que l'on opère à des températures de 205 à 240 °C.

4. Procédé selon les revendications 1 à 3, caractérisé en ce que l'on utilise les sels en solution aqueuse à une concentration de 0,3 à 10 moles/l.

5. Procédé selon les revendications 1 à 4, caractérisé en ce que l'on utilise les aldéhydes de formule R—$CH_2$—CHO sous forme de leurs acétals.